# EUROPÄISCHE PATENTSCHRIFT

(11) **EP 0 938 464 B1**
(45) Veröffentlichungstag und Bekanntmachung des Hinweises auf die Patenterteilung: **31.07.2002**
(21) Anmeldenummer: 97940048.8
(22) Anmeldetag: 30.07.1997
(51) Int. Cl.: C07C 53/21, C07C 51/42, C07C 51/41

(54) **RÜCKGEWINNUNG HOCHFLUORIERTER CARBONSÄUREN AUS DER GASPHASE**
RECOVERY OF HIGHLY FLUORINATED CARBOXYLIC ACIDS FROM THE GASEOUS PHASE
RECUPERATION D'ACIDES CARBOXYLIQUES TRES FLUORES CONTENUS DANS LA PHASE GAZEUSE

(30) Priorität: 05.08.1996 DE 19631406
(43) Veröffentlichungstag der Anmeldung: 01.09.1999
(73) Patentinhaber: Dyneon GmbH & Co. KG, 84504 Burgkirchen (DE); E.I. DU PONT DE NEMOURS AND COMPANY, Wilmington Delaware 19898 (US)
(72) Erfinder: GRASBERGER, Rainer, D-84518 Garching (DE); SULZBACH, Reinhard, Albert, D-84489 Burghausen (DE); BRANDENBURG, Rik, A., NL-4201 BC Gorinchem (NL)
(74) Vertreter: Klein, Otto, Dr.
(86) Internationale Anmeldenummer: EP9704146
(87) Internationale Veröffentlichungsnummer: WO9805621

(56) Entgegenhaltungen:
- DE-A- 2 432 473
- US-A- 4 060 535

## Beschreibung

Das deutsche Patent 195 27 276 (Patentschrift veröffentlicht am 08.08.1996) betrifft ein Verfahren zur Rückgewinnung hochfluorierter Carbonsäuren aus Abgasströmen, das dadurch gekennzeichnet ist, daß man das Abgas mit einer alkalischen Waschlösung der Dichte > 1,15 g/cm³ in Kontakt bringt, so daß sich das Salz der hochfluorierten Carbonsäure als separate Phase abscheidet.

In Weiterentwicklung dieses Erfindungsgedankens wurde nun gefunden, daß als alkalische Waschlösung vorteilhaft eine Kaliumcarbonatlösung dient. Die Erfindung betrifft somit ein Verfahren zur Rückgewinnung hochfluorierter Carbonsäuren aus Abgasströmen, wobei man das Abgas mit einer alkalischen Waschlösung der Dichte > 1,15 g/cm³ in Kontakt bringt, so daß sich das Salz der hochfluorierten Carbonsäure als separate Phase abscheidet, das dadurch gekennzeichnet ist, daß die alkalische Waschlösung eine Kaliumcarbonatlösung ist.

Wenn bei dem Verfahren des deutschen Patents 195 27 276 die alkalische Waschlösung eine Alkalilauge ist, bildet sich das entsprechende Carbonat, wenn der zu reinigende Abgasstrom Kohlendioxid enthält. Beim Einsatz von Natronlauge wurde nun gefunden, daß dieses Natriumcarbonat zu einer Belagsbildung in der Waschkolonne führt. Weiterhin ist diese Carbonatbildung mit einem unerwünschten Verbrauch von Natriumhydroxid verbunden. Diese Nachteile vermeidet der Einsatz von Kaliumcarbonat als alkalisches Mittel.

Wird anstelle von Kaliumcarbonat Natriumcarbonat eingesetzt, so entfällt zwar der Verbrauch von Natriumhydroxid, es kommt aber zu einer stark vermehrten Bildung von Belägen. Weiterhin zeigt Natriumcarbonat ein ungünstigeres Löseverhalten. Das zeigt sich darin, daß die Abkühlung von Lösungen mit der für das Verfahren erforderlichen Dichte bei Natriumcarbonat zu Abscheidungen durch Übersättigung führt. Dies ist bei Kaliumcarbonat nicht der Fall, so daß auf eine Beheizung von Rohrleitungen und Behältern verzichtet werden kann.

Es wurde weiterhin gefunden, daß beim Einsatz von Kaliumcarbonat als alkalisches Mittel das Kaliumsalz der hochfluorierten Carbonsäure in einer Form abgeschieden wird, die leichter filtrierbar ist als beim Einsatz von Kalilauge. Hinzu kommt der bereits erwähnte Vorteil, daß beim Einsatz von Kaliumcarbonat kein Verbrauch an alkalischem Mittel durch absorbiertes Kohlendioxid auftritt.

Die Dichte der alkalischen Waschlösung beträgt vorteilhaft 1,2 bis 1,4 g/cm³.

Bezüglich der weiteren Einzelheiten wird auf das Hauptpatent DE-C-19527276 verwiesen.

Die Erfindung wird nun anhand des folgenden Beispiels noch näher erläutert.

### Beispiel

In eine handelsübliche Waschkolonne mit einer Länge von 2000 mm und einem Innendurchmesser von 250 mm werden 400 Nm³/h Abluft mit einer Temperatur von 171 °C aus einem Trocknungsprozeß für Fluorpolymerpulver eingetragen. Die Abluft enthält 750 mg/Nm³, entsprechend 300 g/h, Perfluoroctansäure. Mit der Abluft werden zusätzlich circa 20 kg/h Wasser aus dem Trockner in den Waschprozeß transportiert. Der Druck in der Waschkolonne beträgt circa 1 bar absolut.

Dem Wäscher werden über eine Düse 10 m³/h einer alkalischen Waschflüssigkeit, bestehend aus wäßriger Kaliumcarbonatlösung mit einer Dichte von 1,30 g/cm³ und einer Temperatur von 45 °C, aufgegeben. Am unteren Teil der Waschkolonne entweicht der gereinigte Abgasstrom von 400 Nm³/h, der nur noch 0,8 mg/Nm³, entsprechend 0,32 g/h Perfluoroctansäure enthält. Zusätzlich ist Wasserdampf, entsprechend dem Wasserpartialdruck, bei der im Wäscher herrschenden Temperatur von circa 45 °C vorhanden.

Der Wäscher wird im Gleichstrom betrieben. Das alkalische Waschmedium und das entstandene Kaliumsalz der Perfluoroctansäure strömen aus der Kolonne direkt in einen Abscheidebehälter mit einem Volumen von 0,4 m³. Dort schwimmt das in der alkalischen Waschlösung nicht lösliche Kaliumsalz der Perfluoroctansäure als breiige Schicht auf. Die alkalische Waschlösung, die praktisch kein Kaliumsalz der Perfluoroctansäure mehr enthält, wird unten aus dem Abscheidebehälter abgezogen und mit einer Pumpe der Waschkolonne erneut zugeführt. Die Konzentration an gelöstem Kaliumsalz der Perfluoroctansäure im Waschmedium beträgt etwa 170 mg/l. Die Dichte der Waschflüssigkeit wird durch Zugabe von Kaliumcarbonat auf dem gewünschten Wert von etwa 1,30 g/cm³ aufrechterhalten. Das als breiige Schicht abgeschiedene Kaliumsalz der Perfluoroctansäure läuft an einem Überlauf des Abscheidebehälters zusammen mit etwas Waschmedium in einen Tank ab. Der Austragsvorgang wird durch einen sehr langsam laufenden Rührer unterstützt, der gerade in die obere Schicht eintaucht. Die Separation der Waschlösung und des sich bildenden Kaliumperfluoroctanoatschaums im Abscheidebehälter ist sehr gut.

In dem Tank scheiden sich nach mehrstündigem Stehen nochmals zwei Phasen ab, eine Unterphase, die im wesentlichen aus überschüssiger Waschflüssigkeit besteht, und eine breiige Oberphase. Die Unterphase wird abgetrennt und in den Waschprozeß zurückgeführt.

Die breiige Oberphase enthält 36 Gew.-% Kaliumperfluoroctanoat.

Das so gewonnene Konzentrat wird der weiteren Aufarbeitung zur Gewinnung reiner 100gew.%iger Perfluoroctansäure zugeführt.

Es sei erwähnt, daß das so gewonnene Konzentrat zwar eine rühr- und pumpfähige Mischung, aber keine stabile Lösung des Kaliumsalzes der Perfluoroctansäure darstellt. Um eine stabile Lösung zu erhalten, müßte noch mehr Wasser zugesetzt werden. Dies ist für die weitere Aufarbeitung jedoch nicht unbedingt erforderlich.

Durch die beschriebene Rückführung der abgetrennten Unterphase in den Waschprozeß geht keine Perfluoroctansäure verloren. Es wird keine Beeinträchtigung des Waschprozesses durch Belagsbildung in der Kolonne bei mehrwöchigem Betrieb beobachtet.

## Patentansprüche

1. Verfahren zur Rückgewinnung hochfluorierter Carbonsäuren aus Abgasströmen, wobei man das Abgas mit einer alkalischen Waschlösung der Dichte > 1,15 g/cm³ in Kontakt bringt, so daß sich das Salz der hochfluorierten Carbonsäure als separate Phase abscheidet, **dadurch gekennzeichnet, daß** die alkalische Waschlösung eine Kaliumcarbonatlösung ist.

## Claims

1. A process for the recovery of highly fluorinated carboxylic acids from off-gas streams, in which the off-gas is brought into contact with an alkaline washing solution of density >1.15 g/cm³ so that the salt of the highly fluorinated carboxylic acid settles out as a separate phase, wherein the alkaline washing solution used is a potassium carbonate solution.

## Revendications

1. Procédé pour la récupération d'acides carboxyliques hautement fluorés à partir de courants de gaz d'échappement, dans lequel on met en contact le gaz d'échappement avec une solution de lavage alcaline de masse volumique > 1,15 g/cm³, de sorte que le sel de l'acide carboxylique hautement fluoré se sépare sous la forme d'une phase distincte, **caractérisé en ce que** la solution de lavage alcaline est une solution de carbonate de potassium.
